# EUROPÄISCHE PATENTSCHRIFT

(11) **EP 2 443 088 B1**
(45) Veröffentlichungstag und Bekanntmachung des Hinweises auf die Patenterteilung: **14.08.2013**
(21) Anmeldenummer: 10725162.1
(22) Anmeldetag: 14.06.2010
(51) Int. Cl.: C07D 207/327, C07C 211/14

(54) **METHYL-SUBSTITUIERTE TETA-VERBINDUNGEN**
METHYL-SUBSTITUTED TETA COMPOUNDS
COMPOSÉS TETA À SUBSTITUTION MÉTHYLE

(30) Priorität: 18.06.2009 EP 09163055
(43) Veröffentlichungstag der Anmeldung: 25.04.2012
(73) Patentinhaber: BASF SE, 67056 Ludwigshafen (DE)
(72) Erfinder: HUGO, Randolf, 67246 Dirmstein (DE); MELDER, Johann-Peter, 67459 Boehl-Iggelheim (DE); BAUMANN, Robert, 68259 Mannheim (DE); OFTRING, Alfred, 67098 Bad Duerkheim (DE); BUSCHHAUS, Boris, 68161 Mannheim (DE); BRASCHE, Gordon, 60594 Frankfurt (DE); AHRENS, Sebastian, 69168 Wiesloch (DE); PFAB, Peter, 67433 Neustadt (DE)
(86) Internationale Anmeldenummer: PCT/EP2010/058287
(87) Internationale Veröffentlichungsnummer: WO 2010/146009

(56) Entgegenhaltungen:
- DE-B- 1 148 866
- US-A- 2 743 243
- US-A- 2 828 276
- US-A- 2 839 480
- US-A- 3 026 203
- US-A- 3 240 664
- US-A- 3 825 521
- US-A- 4 044 053
- US-A1- 2006 041 170
- DATABASE REGISTRY [Online] CHEMICAL ABSTRACTS SERVICE, COLUMBUS, OHIO, US; 16. November 1984 (1984-11-16), XP002598988 Database accession no. RN:65379-59-1
- DATABASE BEILSTEIN [Online] BEILSTEIN INSTITUTE FOR ORGANIC CHEMISTRY, FRANKFURT-MAIN, DE; 1966, XP002598989 Database accession no. RID:4054501
- DATABASE WPI Week 200827 Thomson Scientific, London, GB; AN 2008-D75610 XP002599095 & JP 2008 056849 A (TOSOH CORP) 13. März 2008 (2008-03-13)
- DATABASE REGISTRY [Online] CHEMICAL ABSTRACTS SERVICE, COLUMBUS, OHIO, US; 1. Oktober 2004 (2004-10-01), XP002599096 Database accession no. RN:755713-79-2

## Beschreibung

Die Erfindung betrifft ein Verfahren zur Herstellung von Triethylentetraamin, das mit mindestens einer Methylgruppe substituiert ist (Me-TETA beziehungsweise Methyl-substituierte TETA-Verbindungen). Me-TETA wird hergestellt durch Hydrierung von Biscyanomethylimidazolidin (BCMI) in Gegenwart eines Katalysators. Weiterhin betrifft die vorliegende Erfindung Methyl-substituierte TETA-Verbindungen als solche. Weiterhin betrifft die vorliegende Erfindung die Verwendung von Methyl-substituierten TETA-Verbindungen als Edukt beziehungsweise Zwischenprodukt bei der Herstellung von beispielsweise Beschichtungen oder Haftmitteln.

Die Herstellung von (unsubstituierten) Triethylentetraamin (TETA) ist generell bekannt. TETA gehört zu der Gruppe der Ethylenamine, deren bekannteste Vertreter die kurzkettigen Ethylendiamin (EDA) und Diethylentriamin (DETA) sind. Ethylenamine eignen sich als Ausgangsmaterialien für die Herstellung von zahlreichen Endprodukten unterschiedlicher Industriezweige, beispielsweise im Epoxy-Bereich. So eignen sich Ethylenamine zur Herstellung von Beschichtungen, Haftmitteln, Haftvermittlern, Plastik oder Kunststoffen. Die chemische Zusammensetzung sowie die Eigenschaften der so hergestellten Endprodukte werden stark von der Auswahl des Ethylenamins als Edukt beziehungsweise Zwischenprodukt für die Herstellung solcher Endprodukte beeinflusst.

TETA fällt unter anderem als Nebenprodukt bei der Herstellung von kurzkettigen Ethylenaminen wie EDA und DETA an. So betrifft EP-A 222 934 ein Verfahren zur Herstellung von höheren Alkylenpolyaminen durch Umsetzung eines vicinalen Dihaloalkans mit einem Überschuss an Ammoniak in wässrige Phase unter Zugabe einer starken Base, wobei ein Imin-Zwischenprodukt gebildet wird, das anschließend mit einem Alkylenpolyamin unter Ausbildung des höheren Alkylenpolyamins umgesetzt wird. Als vicinales Dihaloalkan eignet sich insbesondere Ethylendichlorid (EDC oder 1,2-Dichlorethan). Als Alkylenpolyamine werden insbesondere Ethylendiamin oder höhere Ethylenamine wie DETA, aber auch TETA und Tetraethylenpentaamin (TEPA) eingesetzt. Bei diesen Verfahren (EDC-Verfahren) fällt ein Gemisch verschiedener Ethylenamine (lineare Ethylenamine wie EDA, DETA, TETA, TEPA oder höhere Ethylenamine sowie cyclische Derivate wie Piperazin (Pip) oder Aminoethyl-Piperazin (AEPip)) an. Je nachdem, welches Ethylenamin zu den Edukten EDC und NH₃ zugegeben wird, enthält das Reaktionsgemisch einen entsprechenden Anteil an höheren Ethylenaminen.

Darüber hinaus gibt es auch gezielte Verfahren zur Herstellung von TETA. Ein solches Verfahren ist in WO 2008/104582 beschrieben, wonach TETA durch Hydrierung von Ethylendiamindiacetonitril (EDDN) an einem Katalysator hergestellt wird. EDDN wiederum ist erhältlich durch Umsetzung von EDA mit Formaldehyd und Blausäure (HCN). Die Umsetzung von EDA mit Formaldehyd und Blausäure kann in unterschiedlichen Varianten durchgeführt werden, beispielsweise unter Ausbildung des Zwischenprodukts Formaldehydcyanhydrin (FACH), indem zunächst Formaldehyd und Blausäure in Abwesenheit von EDA umgesetzt werden. Je nach Wahl der Eduktkonzentrationen kann neben EDDN zusätzlich die entsprechende Mononitril-Verbindung Ethylendiaminmonoacetonitril (EDMN) gebildet werden. Die Herstellung von TETA durch direkte Hydrierung von EDDN ist im nachfolgenden Schema 1 dargestellt:

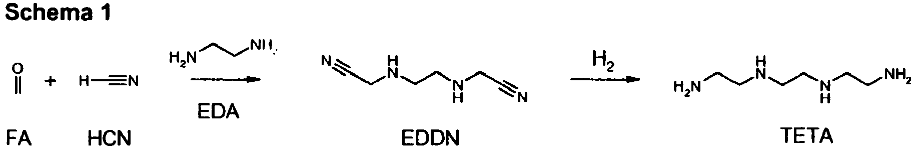

Ein alternatives Verfahren zur direkten Hydrierung von EDDN ist in US-A 2006/0041170 beschrieben, wonach vor der Hydrierung unterschiedliche Schutzgruppen auf die beiden Aminofunktionen von EDDN aufgezogen werden. Im Anschluss an die Hydrierung werden die Schutzgruppen wiederum entfernt unter Ausbildung von (unsubstituierten) TETA-Salzen. So sind zwei unterschiedliche Schutzgruppenverfahren unter Verwendung von Benzaldehyd offenbart, in denen kein Wasserstoff, sondern Lithiumaluminiumhydrid eingesetzt wird. In einem dritten Verfahren wird die Hydrierung mit Wasserstoff in Gegenwart einer Boc-Schutzgruppe durchgeführt. Nachteilig an dem in US-A 2006/0041170 beschriebenen Verfahren ist insbesondere, dass es sich um ein mehrstufiges Hydrierverfahren handelt, bei dem das eingesetzte Edukt EDDN zuerst chemisch derivatisiert werden muss (Schutzgruppen), um die Hydrierung durchzuführen. Im Anschluss an die Hydrierung müssen die Schutzgruppen in mehreren Reaktionsschritten wieder abgespalten werden, wobei zunächst (unsubstituiertes) TETA als Salz und nicht in der freien Basenform anfällt.

US3026203 beschreibt die Herstellung von N, N'-dimethyltriethylenetetramine durch Reduktion eines Di (methylamino)-diamid

Die der vorliegenden Erfindung zu Grunde liegende Aufgabe besteht in der Bereitstellung von neuen TETA-Derivaten, die über mindestens einen Methylsubstituenten verfügen sowie ein Verfahren zur Herstellung dieser neuen TETA-Derivate. Auf diese neuen Methyl-substituierten TETA-Verbindungen können somit auch neue Folgeprodukte hergestellt werden, die über modifizierte Anwendungseigenschaften verfügen.

Die Aufgabe wird gelöst durch ein Verfahren zur Herstellung von Triethylentetraamin, das mit mindestens einer Methylgruppe substituiert ist (Me-TETA), umfassend die Hydrierung von Biscyanomethylimidazolidin (BCMI) in Gegenwart eines Katalysators.

Das erfindungsgemäße Verfahren hat den Vorteil, dass damit gezielt eine neue Substanzklasse von TETA-Derivaten (also Methyl-substituierte TETA-Verbindungen/Me-TETA) hergestellt werden können. Me-TETA fällt dabei mit hohem Umsatz und/oder hoher Selektivität an. Mit den erfindungsgemäßen Methyl-substituierten TETA-Verbindungen können wiederum Folgeprodukte mit einer neuen chemischen Zusammensetzung und somit auch modifizierten Eigenschaften gezielt hergestellt werden.

Unter einem Triethylentetraamin, das mit mindestens einer Methylgruppe substituiert ist (Me-TETA), wird im Rahmen der vorliegenden Verbindung jedes Triethylentetraamin (TETA)-Derivat verstanden, in dem ein, zwei oder mehrere der Wasserstoffatome, die an die vier Aminofunktionen des unsubstituierten TETA gebunden sind, durch die entsprechende Anzahl an Methylgruppen (CH₃-) substituiert sind.

Me-TETAs gemäß der vorliegenden Erfindung sind im nachfolgenden Schema 2 als Verbindungen (2) bis (13) beispielhaft aufgeführt.

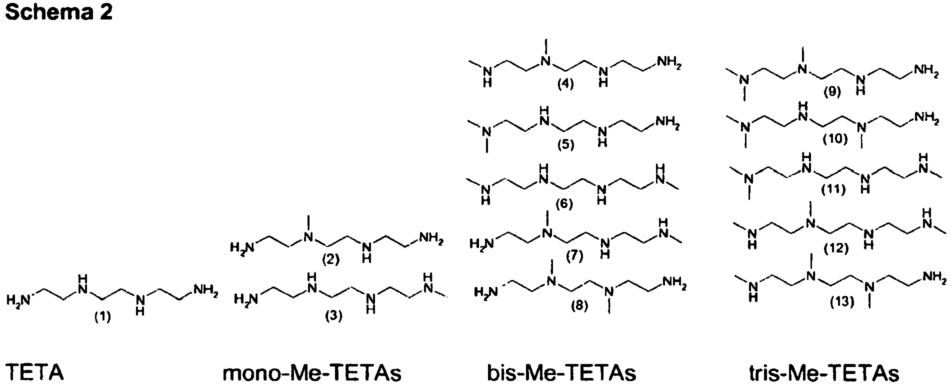

In Abhängigkeit von den gewählten Hydrierbedingungen wie Druck, Temperatur oder Katalysator, können im erfindungsgemäßen Verfahren bei der Hydrierung von BCMI Me-TETAs mit einer unterschiedlichen Anzahl an Methylgruppen gebildet werden. Darüber hinaus fällt als Nebenprodukt auch unsubstituiertes TETA (Schema 2: Verbindung 1) an. Wie Schema 2 zu entnehmen ist, können im erfindungsgemäßen Verfahren Me-TETAs mit einer (Mono-Me-TETA; Verbindungen 2 und 3), zwei (Bis-Me-TETA; Verbindungen 4 bis 8) und drei Methylsubstituenten (tris-Me-TETA; Verbindungen 9 bis 13) gebildet werden. Darüber hinaus können auch Me-TETAs, bei denen vier, fünf oder alle sechs Wasserstoffatome des unsubstituierten TETAs mit Methylgruppen substituiert sind, als Nebenprodukte gebildet werden.

Bei der Hydrierung von BCMI im erfindungsgemäßen Verfahren fällt in der Regel ein Gemisch aus mindestens zwei Me-TETAs gemäß vorstehendem Schema 2 an. Das Verhältnis der einzelnen Me-TETA-Verbindungen ist variabel, da es von den sonstigen Hydrierparametem wie Druck, Temperatur oder Katalysator stark beeinflusst wird. Die Hydrierparameter können so gewählt werden, dass selektiv eines oder mehrere der in Schema 2 aufgeführten Me-TETAs hergestellt werden. Dies bedeutet wiederum, dass je nach Wahl der Hydrierparameter einige der in Schema 2 aufgeführten Me-TETAs gar nicht gebildet werden, sofern gezielt eines oder mehrere der übrigen Me-TETAs hergestellt wird. Sofern im erfindungsgemäßen Verfahren bei der Hydrierung von BCMI ein Gemisch enthaltend mindestens 2 Me-TETAs anfällt, können die einzelnen Me-TETAs nach dem Fachmann bekannten Methoden voneinander getrennt beziehungsweise aus dem Me-TETA-Gemisch isoliert werden.

Gegenstand der vorliegenden Erfindung ist somit das nachfolgend näher beschriebene Verfahren zur Herstellung von Triethylentetraamin, das mit mindestens einer Methylgruppe substituiert ist (Me-TETA). Vorzugsweise ist das Me-TETA ausgewählt aus Triethylentetraamin, das mit einer Methylgruppe substituiert ist (mono-Me-TETA), Triethylentetraamin, das mit zwei Methylgruppen substituiert ist (bis-Me-TETA) oder Triethylentetraamin, das mit drei Methylgruppen (tris-Me-TETA) substituiert ist.

Besonders bevorzugt ist Me-TETA ein mono-Me-TETA. Insbesondere ist das Me-TETA ausgewählt aus N-2-Aminoethyl-N'-(2-N"-methylaminoethyl)-1,2-ethandiamin (sek-Me-TETA) oder N-2-Aminoethyl-N-methyl-N'-2-aminoethyl-1,2-ethandiamin (tert-Me-TETA). Sek-Me-TETA und tert-Me-TETA sind in Schema 2 als Verbindungen 2 beziehungsweise 3 abgebildet.

Hydrieren (Hydrierung) im Rahmen der vorliegenden Erfindung bedeutet die Reaktion des Aminonitrils BCMI mit Wasserstoff. Verfahren zur Herstellung von Biscyanomethylimidazolidin (BCMI) sind dem Fachmann prinzipiell bekannt. Vorzugsweise wird BCMI hergestellt durch Umsetzung von Ethylendiamindiacetonitril (EDDN) und Formaldehyd. Vorzugsweise erfolgt diese Umsetzung in Batch-Fahrweise. Gegebenenfalls kann BCMI auch in kontinuierlicher Fahrweise hergestellt werden, beispielsweise mit einem Überschuss an Formaldehyd. Hierbei kann Wasser als Lösungsmittel verwendet werden, eine typische Temperatur ist 60°C sowie eine Verweilzeit von 6 Minuten. Vorzugsweise kann das im BCMI-haltigen Reaktionsgemisch enthaltene Wasser durch eine Destillation abgetrennt werden. Zur Durchführung einer solchen Destillation können neben Füllkörper- oder Bodenkolonnen auch Dünnschichtverdampfer (Dünnfilmverdampfer) eingesetzt werden. Vorzugsweise erfolgt die Destillation von Wasser im einem Dünnschichtverdampfer. Geeignete Dünnschichtverdampfer sind dem Fachmann bekannt (siehe auch Ullmanns Enzyklopädie der technischen Chemie, Band 2, vierte Auflage, Verlag Chemie, Weinheim (1972), Seite 656-657). Bevorzugte Dünnschichtverdampfer sind vom Typ "Sambay", "Luwa" oder "Sako", besonders bevorzugt ist ein Sambay-Dünnschichtverdampfer. Gegebenenfalls können zur Abtrennung von Wasser aus dem BCMI-haltigen Reaktionsgemisch außer der Destillation noch weitere Wasserabtrennungsschritte wie Extraktion, Trocknung, Filtration etc. durchgeführt werden.

Als Katalysatoren zur Hydrierung der beiden Nitril-Funktionen vom BCMI zum Amin können Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten. In einer bevorzugten Ausführungsform werden im erfindungsgemäßen Verfahren Raney-Katalysatoren eingesetzt, bevorzugt Raney-Kobalt- oder Raney-Nickel-Katalysatoren und besonders bevorzugt mit mindestens einem der Elemente Cr, Ni oder Fe dotierte Raney-Kobalt- oder mit einem der Elemente Mo, Cr oder Fe dotierte Raney-Nickel-Katalysatoren.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff enthaltendem Gasstrom bei erhöhter Temperatur aktiviert. Wenn die Katalysatoren außerhalb des Reaktors reduziert werden, kann danach eine Passivierung durch einen Sauerstoff enthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie zum Beispiel. in EP-A 1 209 146 beschrieben, aktiviert werden können.

Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂,1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ beziehungsweise MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ beziehungsweise MnO₂ enthält. Beispielsweise der in dieser Schrift konkret offenbarte Katalysator mit der Zusammensetzung 31,5 Gew.-% ZrO₂, 50 Gew.-% NiO, 17 Gew.-% CuO und 1,5 Gew.-% MoO₃. Ebenso geeignet sind die in WO-A 99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als 'Metallschwamm' aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach zum Beispiel mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typischerweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie zum Beispiel Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

In einer bevorzugten Ausführungsform wird erfindungsgemäß ein Raney-Kobalt-Skelett-Katalysator eingesetzt, der aus einer Co/Al-Legierung durch Laugung mit wässriger Alkalimetallhydroxid-Lösung, zum Beispiel Natronlauge, und nachfolgender Waschung mit Wasser erhalten wurde, und bevorzugt als Promotoren mindestens eines der Elemente Fe, Ni oder Cr enthält.

Solche Katalysatoren enthalten typischerweise neben Kobalt noch 1 bis 30 Gew.-% Al, besonders 2 bis 1.2 Gew.-% Al, ganz besonders 3 bis 6 Gew.-% Al, 0 bis 10 Gew.-% Cr, besonders 0,1 bis 7 Gew.-% Cr, ganz besonders 0,5 bis 5 Gew.-% Cr, insbesondere 1,5 bis 3,5 Gew.-% Cr, 0 bis 10 Gew.-% Fe, besonders 0,1 bis 3 Gew.-% Fe, ganz besonders 0,2 bis 1 Gew.-% Fe, und/oder 0 bis 10 Gew.-% Ni, besonders 0,1 bis 7 Gew.-% Ni, ganz besonders 0,5 bis 5 Gew.-% Ni, insbesondere 1 bis 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:

Al: 2 bis 6 Gew.-%, Co: ≥ 86 Gew.-%, Fe: 0 bis 1 Gew.-%, Ni: 1 bis 4 Gew.-%, Cr: 1,5 bis 3,5 Gew.-%.

Ebenfalls kann erfindungsgemäß ein Nickel-Skelett-Katalysator eingesetzt werden, der aus einer Ni/Al-Legierung durch Laugung mit wässriger Alkalimetallhydroxid-Lösung, zum Beispiel Natronlauge, und nachfolgender Waschung mit Wasser erhalten wurde, und bevorzugt als Promotoren mindestens eines der Elemente Fe, Cr enthält.

Solche Katalysatoren enthalten typischerweise neben Nickel noch 1 bis 30 Gew.-% Al, besonders 2 bis 20 Gew.-% Al, ganz besonders 5 bis 14 Gew.-% Al,
0 bis 10 Gew.-% Cr, besonders 0,1 bis- 7 Gew.-% Cr, ganz besonders 1 bis 4 Gew.-% Cr,
und/oder
0 bis 10 Gew.-% Fe, besonders 0,1 bis- 7 Gew.-% Fe, ganz besonders 1 bis 4 Gew.-% Fe,
wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden. Dieser Katalysator weist folgende Zusammensetzung auf

Al: ≤14 Gew.-%, Ni: ≥ 80 Gew.-%, Fe: 1 bis 4 Gew.-%, Cr: 1 bis 4 Gew.-%.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

Die Temperaturen, bei denen die Hydrierung durchgeführt wird, liegen in einem Bereich von 40 bis 150°C, bevorzugt von 70 bis 140°C, insbesondere bei 80 bis 140°C.

Der bei der Hydrierung herrschende Druck liegt im Allgemeinen bei 5 bis 300 bar, bevorzugt bei 30 bis 250 bar, besonders bevorzugt bei 40 bis 160 bar.

In einer bevorzugten Ausführungsform wird BCMI mit einer Rate der Hydrierung zugeführt, die nicht größer ist als die Rate, mit der BCMI mit Wasserstoff bei der Hydrierung reagiert. Die Zuführrate ist bevorzugt so einzustellen, dass quasi Vollumsatz erreicht wird. Dieses wird durch Temperatur, Druck, Art des Gemisches, Menge und Art des Katalysators, des Reaktionsmediums, Durchmischungsgüte des Reaktorinhalts, Verweilzeit etc. beeinflusst.

Die Hydrierung wird vorzugsweise in Gegenwart von einem Lösungsmittel, beispielsweise einem organischen Lösungsmittel durchgeführt. Gegebenenfalls kann die Hydrierung auch in Gegenwart von Wasser durchgeführt werden, da eine vollständige Wasserabtrennung vor der Hydrierung nicht notwendig ist. Beispielsweise kann die Hydrierung in einem organischen Lösungsmittel durchgeführt werden, das Wasser in Spuren oder als Nebenkomponente enthält. Die Verwendung von Wasser als Lösungsmittel alleine oder von Lösungsmittelgemischen mit einem Wasserüberschuss ist zwar möglich, aber weniger geeignet als die Verwendung eines organischen Lösungsmittels alleine oder im Überschuss.

Ein geeignetes Lösungsmittel, welches ein oder mehrere Komponenten umfassen kann, sollte bevorzugt folgende Eigenschaften aufweisen:
(a) das Lösungsmittel sollte stabilisierend auf BCMI wirken, insbesondere deren Zersetzung bei den herrschenden Temperaturen verhindern;
(b) das Lösungsmittel sollte eine gute Wasserstofflöslichkeit zeigen;
(c) das Lösungsmittel sollte bei den Reaktionsbedingungen inert sein;
(d) das Reaktionsgemisch (BCMI, gegebenenfalls Wasser sowie Lösungsmittel) sollte unter Reaktionsbedingungen einphasig sein;
(e) die Lösemittelauswahl sollte im Hinblick auf eine bevorzugt destillative Abtrennung des Produktes im Anschluss an die Hydrierung aus dem Produktstrom erfolgen. Wobei energie- oder apparativ-aufwändige (zum Beispiel engsiedende Gemische oder schwierig zu trennende Azeotrope) Trennungen zu vermeiden sind.
(f) das Lösungsmittel sollte gut von den Produkten abtrennbar sein, d.h. die Siedetemperatur sollte sich hinreichend von der der Produkte unterscheiden. Hierbei wird eine niedrigere Siedetemperatur als die der Produkte bevorzugt.

Bevorzugte Lösungsmittel sind organische Lösungsmittel, beispielsweise Amide, wie N-Methylpyrrolidon (NMP) und Dimethylformamid (DMF), aromatische und aliphatische Kohlenwasserstoffe, wie Benzol, Toluol und Xylol, Alkohole wie Methanol, Ethanol, Propanol, Isopropanol, Butanol, Isobutanol, sekundäres Butanol und tertiäres Butanol, Amine, Ester, wie Essigsäuremethylester oder Essigsäureethylester und Ether, wie Diisopropylether, Diisobutylether, Glykoldimethylether, Diglykoldimethylether, Dioxan und Tetrahydrofuran (THF). Bevorzugt werden im erfindungsgemäßen Verfahren Ether verwendet, mehr bevorzugt cyclische Ether und besonders bevorzugt Tetrahydrofuran oder 2-Methyl-Tetrahydrofuran (2-Me-THF). In einer weiteren bevorzugten Ausführungsform werden Alkohole, insbesondere Methanol, als organisches Lösungsmittel verwendet.

Die Verwendung eines organischen Lösungsmittels (inerten organischen Verbindung) erweist sich als vorteilhaft, da dadurch eine Stabilisierung der einzelnen Komponenten des (gegebenenfalls wässrigen) Aminonitrilgemisches, insbesondere in Gegenwart der resultierenden Amine, erzielt werden kann. Außerdem lässt sich durch die Verwendung von organischen Lösungsmitteln ein Spüleffekt (Reduzierung der Spülzyklen, Verminderung der Katalysatorausschleusung) an dem eingesetzten Katalysator erzielen, wodurch dessen Standzeit erhöht beziehungsweise dessen Verbrauch erniedrigt (längere Katalysatorlebensdauer) sowie die Katalysatorbelastung verbessert werden kann. Durch die Verwendung geeigneter Lösungsmittel kann weiterhin die Bildung von weiteren Nebenprodukten wie AEPip vermindert werden. ,

Das Lösungsmittel wird im Gewichtsverhältnis zu dem eingesetzten Aminonitril (BCMI) von 0,1 : 1 bis 15 : 1 eingesetzt. Die Konzentration des Aminonitrilgemisches in der Lösung, in der die Hydrierung durchgeführt wird, sollte so gewählt werden, dass eine geeignete Zuführrate beziehungsweise Verweilzeit eingestellt werden kann. Es ist bevorzugt, das Aminonitril zu 5 bis 50 Gew.-% mit dem Lösungsmittel zu vermischen. Bezogen auf die besonders bevorzugten Lösungsmittel Methanol beziehungsweise THF oder 2-Me-THF ist es beispielsweise vorteilhaft, das Aminonitril zu 20 bis 40 Gew.-% bezogen auf das Lösungsmittel einzusetzen.

Der Anteil an Wasser in der Lösung liegt normalerweise in einem Bereich von 0 bis 50 Gew.-%, vorzugsweise bei 0 bis 30 Gew.-%, insbesondere ≤ 5 Gew.-%. Besonders bevorzugt wird die Hydrierung wasserfrei durchgeführt (Wassergehalt ≤ 0.1 Gew.-%). Die Mengenangaben des Wassers beziehen sich dabei auf das Aminonitril.

Gegebenenfalls können in der Lösung, in der die Hydrierung durchgeführt wird, zusätzliche Additive enthalten sein. Als Additive kommen prinzipiell Hydroxide wie Alkalimetallhydroxide, Alkoholate, Amide, Amine in Frage. Vorzugsweise eignen sich als Additive Amine, besonders EDA und Ammoniak, insbesondere EDA. Weiterhin können auch saure Additive, wie zum Beispiel Silikate zusätzlich in der Lösung enthalten sein. Diese Substanzen können als Reinstoff oder gelöst in einem Lösungsmittel zugesetzt werden. Vorzugsweise wird das erfindungsgemäße Verfahren unter Zusatz von Additiven durchgeführt.

In einer Ausführungsform des Verfahrens wird der Lösung, in der die Hydrierung durchgeführt wird, kein Ammoniak zugesetzt. Sofern noch Ammoniak in den Edukten beziehungsweise in der gegebenenfalls eingesetzten wässrigen Lösung gelöst ist beziehungsweise als Nebenprodukt bei der Hydrierung freigesetzt wird, ist dies nicht störend. Gegebenenfalls vorhandener Ammoniak kann nach dem Fachmann bekannten Methoden, beispielsweise destillativ, entfernt werden. Sofern auf Ammoniak verzichtet wird, hat dies den Vorteil, dass der Eigendruck des Systems verringert ist.

Im erfindungsgemäßen Verfahren können ein (oder mehrere) Lösungsmittel verwendet werden, wobei das Lösungsmittel zunächst mit BCMI vermischt wird. Die erhaltene Lösung, die gegebenenfalls auch Additive enthalten kann, wird anschließend in das den Katalysator enthaltende Reaktionsgefäß zugeführt. Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird. Bei kontinuierlichen Verfahren kann eine Teilmenge des Lösungsmittels auch separat von der Lösung, die BCMI, das Lösungsmittel und gegebenenfalls das Additiv enthält, in das Reaktionsgefäß zugegeben werden. In einer bevorzugten Ausführungsform erfolgt die Zuführung des in der Lösung enthaltenen BCMIs mit einer Rate, die nicht größer ist als die Rate, mit der BCMI mit Wasserstoff bei der Hydrierung reagiert. Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird.

In einer bevorzugten Ausführungsform der vorliegenden Erfindung erfolgt die Hydrierung von BCMI im Semibatch-Verfahren mit einem Raney-Cobalt-Katalysator. Vorzugsweise wird als Lösungsmittel 2-Me-THF eingesetzt, die Temperatur beträgt 120°C und der Druck 100 bar. Der Katalysator wird dabei in 2-Me-THF vorgelegt und das Aminonitril (BCMI) in 2h als 18%-ige Lösung zudosiert.

Das erfindungsgemäße Verfahren zur Herstellung von Me-TETA durch Hydrierung von BCMI kann in üblichen für die Katalyse geeigneten Reaktionsgefäßen in einer Festbett-, Wirbelschicht-, oder Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des Aminonitrils und des Katalysators mit dem gasförmigen Wasserstoff unter Druck möglich ist.

Die Hydrierung in Suspensionsfahrweise kann in einem Rührreaktor, Strahlschlaufenreaktor, Strahldüsenreaktor, Blasensäulenreaktor, beziehungsweise in einer Kaskade derartiger gleicher oder verschiedener Reaktoren durchgeführt werden. Für die Hydrierung an einem Festbettkatalysator sind Rohrreaktoren aber auch Rohrbündelreaktoren denkbar.

Im Fall eines Festbettkatalysators wird in Sumpf- oder Rieselfahrweise mit dem Aminonitril beaufschlagt. Bevorzugt wird allerdings die Suspensionsfahrweise in semikontinuierlicher und bevorzugt in kontinuierlicher Fahrweise eingesetzt.

Die Hydrierung der Nitrilgruppen findet unter Freisetzung von Wärme statt, die in der Regel abgeführt werden muss. Die Wärmeabfuhr kann durch eingebaute Wärmeüberträgerflächen, Kühlmantel oder außenliegende Wärmeüberträger in einem Umlaufkreis um den Reaktor erfolgen. Der Hydrierreaktor beziehungsweise eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen. Insbesondere kann der Kreislaufstrom mittels eines externen Wärmeübertragers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Wie vorstehend bereits ausgeführt, wird im erfindungsgemäßen Verfahren bei der Hydrierung mindestens ein Me-TETA als Hauptprodukt hergestellt. Darüber hinaus können im erfindungsgemäßen Verfahren im Hydrierungs-Reaktionsgemisch auch Nebenprodukte enthalten sein, wie kürzere Ethylenamine (beispielsweise Diethylentriamin/DETA), Hochsieder, cyclische Verbindung (wie AEPip - Aminoethylenpiperazin) oder (unsubstituiertes) TETA als solches. Verfahren zur Abtrennung dieser Nebenprodukte sowie Verfahren zur Auftrennung eines Me-TETA-Gemisches, das eines oder mehrere der in Schema 2 dargestellten Me-TETAs enthält, sind dem Fachmann bekannt. Die Nebenproduktabtrennung und/oder die Auftrennung eines Me-TETA-Gemischs kann beispielsweise durch Destillation oder als chromatographisches Trennverfahren erfolgen. Dies kann gegebenenfalls in Kombination und/oder mehrstufig erfolgen.

Das zur Herstellung von BCMI verwendete EDDN kann prinzipiell nach dem Fachmann bekannten Methoden hergestellt werden, siehe beispielsweise WO 2008/104582. Normalerweise wird EDDN hergestellt durch Umsetzung von EDA mit Formaldehyd und Blausäure (HCN). Vorzugsweise beträgt das molare Verhältnis von EDA zu Formaldehyd zu HCN 1 : 1,5 : 1,5 bis 1 : 2 : 2 [mol/mol/mol].

Sofern nachstehend nicht anders angegeben (Option i) bis iv)), können die Eduktkomponenten von zur EDDN-Herstellung in beliebiger Reihenfolge in das jeweilige Reaktionsgefäß gegeben werden. Beispielsweise kann ein Edukt vollständig vorgelegt werden und ein zweites Edukt zugegeben werden. Vorzugsweise kann EDDN gemäß einer der nachfolgend aufgeführten Optionen i) bis iv) hergestellt werden. Besonders bevorzugt wird EDDN nach Option i) hergestellt.

Gemäß Option i) wird Formaldehyd und HCN zunächst zu Formaldehydcyanhydrin (FACH) umgesetzt, wobei anschließend EDA wiederum mit FACH umgesetzt wird und das molare Verhältnis von EDA zu FACH 1 : 1,5 bis 1 : 2 [mol/mol] beträgt. EDA, Formaldehyd und HCN sind kommerziell erhältliche Produkte oder können prinzipiell nach dem Fachmann bekannten Methoden hergestellt werden. Vorzugsweise wird EDA im erfindungsgemäßen Verfahren in Form seiner freien Base eingesetzt, gegebenenfalls können jedoch auch Salze wie das Dihydrochlorid von EDA als Edukt verwendet werden.

Die Umsetzung von Formaldehyd und HCN ist dem Fachmann bekannt. Die Herstellung von FACH kann durch Umsetzung von wässrigem Formaldehyd mit Blausäure erfolgen. Vorzugsweise liegt Formaldehyd als 30 bis 50 %ige wässrige Lösung vor, Blausäure wird vorzugsweise in 90 bis 100 %iger Reinheit eingesetzt. Diese Umsetzung erfolgt vorzugsweise bei einem pH-Wert von 5,5, der vorzugsweise mit Natronlauge oder Ammoniak eingestellt wird. Die Umsetzung kann bei Temperaturen von 20 bis 70°C beispielsweise im Schlaufen- und/oder Rohrreaktor erfolgen.

Anstelle von aufgereinigter Blausäure (HCN) kann auch HCN-Rohgas in wässriger Formaldehyd-Lösung unter den oben genannten Bedingungen zu FACH chemisobiert werden. Das HCN-Rohgas wird vorzugsweise durch Pyrolyse von Formamid hergestellt und enthält neben Wasser insbesondere geringe Anteile an Ammoniak.

Gegebenenfalls kann die erhaltene wässrige FACH-Lösung durch schonende Vakuumeindämpfung, beispielsweise mit einem Fallfilm- oder Dünnschichtverdampfer, aufkonzentriert und von Leichtsiedern, insbesondere von Blausäure befreit werden. Vorzugsweise erfolgt eine Aufkonzentrierung auf eine 50-80 %ige FACH-Lösung. Vor der Aufkonzentrierung ist eine Stabilisierung der FACH-Lösung durch Erniedrigung des pH-Wertes auf ≤ 4, bevorzugt auf ≤ 3 vorteilhaft, beispielsweise durch Säurezugabe, zum Beispiel durch Zugabe von Phosphorsäure oder vorzugsweise von Schwefelsäure.

Vorzugsweise beträgt in Option i) das molare Verhältnis von EDA zu FACH ungefähr 1 : 1,8 bis 1 : 2 [mol/mol], insbesondere ca. 1 : 2 [mol/mol].

Nach Option ii) wird EDDN durch Umsetzung eines Ethylendiamin-Formaldehyd-Addukt (EDFA) mit Blausäure (HCN) umgesetzt, wobei das molare Verhältnis von ED-FA zu HCN 1 : 1,5 bis 1 : 2 [mol/mol] beträgt. Vorzugsweise beträgt das molare Verhältnis von EDFA zu HCN 1 : 1,8 bis 1 : 2 [mol/mol], insbesondere ca. 1 : 2 [mol/mol]. EDFA wird vorzugsweise durch Vermischen von in etwa äquimolaren Mengen an EDA und Formaldehyd hergestellt.

Gemäß Option iii) wird EDA mit einem Gemisch aus Formaldehyd und Blausäure (GFB) umgesetzt, wobei das molare Verhältnis von EDA zu GFB 1 : 1,5 bis 1 : 2 [mol/mol] beträgt. Vorzugsweise beträgt das molare Verhältnis von EDA zu GFB 1 : 1,8 bis 1 : 2 [mol/mol], insbesondere ca. 1 : 2 [mol/mol]. Vorzugsweise wird das GFB durch Vermischen von in etwa äquimolaren Mengen aus Formaldehyd und Blausäure hergestellt.

Gemäß Option iv) wird EDA mit Formaldehyd und Blausäure (HCN) zeitlich (parallel) umgesetzt, wobei das molare Verhältnis von EDA zu Formaldehyd zu HCN 1 : 1,5 : 1,5 bis 1 : 2 : 2 [mol/mol/mol] beträgt. Vorzugsweise beträgt das molare Verhältnis von EDA zu Formaldehyd zu HCN 1 : 1,8 : 1,8 bis 1 : 2 : 2 [mol/mol/mol], insbesondere ca. 1 : 2 : 2 [mol/mol/mol]. Vorzugsweise werden in dieser Ausführungsform die drei Eduktkomponenten zeitgleich oder schrittweise in gleichen molaren Teilmengen bezogen auf die jeweilige Eduktgesamtmenge in das Reaktionsgefäß zugegeben.

Unter Umständen können die jeweiligen Edukte beziehungsweise Zwischenprodukte direkt im Anschluss an ihre Herstellung im erfindungsgemäßen Verfahren eingesetzt werden. Beispielsweise kann in Option i) FACH ohne vorherige Isolierung im erfindungsgemäßen Verfahren als Edukt eingesetzt werden. Gegebenenfalls kann jedoch FACH im Anschluss an dessen Herstellung zuerst isoliert werden, um anschließend im erfindungsgemäßen Verfahren eingesetzt zu werden.

In einer Ausführungsform der vorliegenden Erfindung wird die EDDN-Herstellung frei oder zumindest im Wesentlichen frei von Cyanosalzen, wie KCN, durchgeführt.

Die EDDN-Herstellung wird normalerweise in Gegenwart eines Lösungsmittels durchgeführt. Vorzugsweise werden im erfindungsgemäßen Verfahren zur Herstellung von EDDN die Edukte in wässriger Phase umgesetzt. Gegebenenfalls können neben Wasser noch weitere, dem Fachmann bekannte Lösungsmittel verwendet werden, die mit Wasser mischbar sind. Weniger bevorzugt werden jedoch Alkohole, insbesondere Methanol als Lösungsmittel verwendet.

Die EDDN-Herstellung wird vorzugsweise bei einer Temperatur von 10 bis 90°C, insbesondere bei 30 bis 70°C durchgeführt. Die Reaktion kann bei Normaldruck oder gegebenenfalls auch bei erhöhtem Druck (Überdruck) durchgeführt werden. Vorzugsweise wird die EDDN-Herstellung in einem Rohrreaktor oder einer Rührkesselkaskade durchgeführt. Vorzugsweise kann die EDDN-Herstellung auch als kontinuierliches Verfahren durchgeführt werden, insbesondere als großtechnisches Verfahren.

Sofern bei der Herstellung von EDDN das entsprechende Mononitril Ethylendiaminmonoacetonitril (EDMN) als Nebenprodukt gebildet wird, kann dieses vorzugsweise im Anschluss an die EDDN-Synthese nach dem Fachmann bekannten Methoden abgetrennt werden. Gegebenenfalls kann aber auch ein Aminonitrilgemisch enthaltend EDDN und EDMN im erfindungsgemäßen Verfahren eingesetzt werden. Die aus EDMN gebildeten Folgeprodukte bei der anschließenden Umsetzung mit Formaldehyd oder der Hydrierung können ebenfalls nach dem Fachmann bekannten Methoden von BCMI beziehungsweise Me-TETA abgetrennt werden. Vorzugsweise erfolgt die EDDN-Herstellung jedoch so, dass ein geringer Anteil an EDMN vorliegt. Vorzugsweise beträgt der Gehalt an EDMN sowie eventuell an noch weiteren Nebenprodukten, beispielsweise sonstigen Aminonitrilen, ≤ 10 Gew.-%, insbesondere ≤ 5 Gew.-%, bezogen auf EDDN.

Im Anschluss an die EDDN-Herstellung oder gegebenenfalls im Anschluss an die BCMI-Herstellung kann im erfindungsgemäßen Verfahren vor der Hydrierung eine Leichtsieder-Abtrennung durchgeführt werden. Sofern zur Herstellung von EDDN FACH verwendet wird, kann die Leichtsiederabtrennung bereits vor der Umsetzung von FACH mit EDA erfolgen. Vorzugsweise werden als Leichtsieder Blausäure (HCN) abgetrennt. HCN kann dabei auch als Zersetzungsprodukt von FACH auftreten. Weiterhin kann an dieser Stelle eventuell von Ammoniak abgetrennt werden. Vorzugsweise erfolgt die Abtrennung durch Destillation, beispielsweise in Form einer Dünnschichtverdampfung wie zum Beispiel einer Sambay-Destillation. Gegebenenfalls kann das Reaktionsgemisch auch mit Stickstoff gestrippt werden.

Außer der Leichtsiederabtrennung kann mit dem EDDN und/oder dem BCMI ein Reinigungsschritt durch Adsorption von Verunreinigungen an einem Absorbens, zum Beispiel Aktivkohle oder Ionenaustauscher, durchgeführt werden. Dies kann zum Beispiel in einer mit dem Absorbens gefüllten Absorptionskolonne erfolgen.

Die nachfolgenden Beispiele verdeutlichen die vorliegende Erfindung.

### Aminonitrilsynthese

### Beispiel 1: BCMI Synthese

Eine Lösung von EDDN (50.0 g, 360 mmol) in Formaldehyd (36.0 g, 360 mmol, 30 Gew.-% in H₂O) in einem Dreihalskolben wird für 15 min bei 90°C gerührt und anschließend im Eisbad abgekühlt. Mittels Sambay wird bei 75°C und 50 mbar das Wasser bis zu einem Restgehalt von 1.5 Gew.-% abgetrennt. Zur weiteren Wasserabtrennung wird das Destillat in CH₂Cl₂ (60 mL) aufgenommen, die Lösung über Na₂SO₄ getrocknet, filtriert und am Rotationsverdampfer eingeengt. Die Zielverbindung BCMI (46.0 g, 307 mmol, 0.1 Gew.-% Restwassergehalt) wird als orangenes Öl analysenrein erhalten.

### Hydrierung

### Generelles:

Die Hydrierung erfolgt in 2-Me-THF als Lösungsmittel bei 120°C und 100bar im Semibatch-Verfahren. Dazu wird der Katalysator Raney-Cobalt 2724 der Firma Grace Division in 2-Me-THF vorgelegt und eine Lösung der jeweiligen Aminonitrile in 2-Me-THF über 120 min zudosiert und für weitere 60 min nachgerührt. Für die Auswertung der Versuche wird ein interner Standard (DEGDME) mit zudosiert. Zur Analyse wird der zweiphasige Reaktionsaustrag mit Methanol homogenisiert. Die Auswertung erfolgt in Gew.-%.

### Analysenmethode zur Identifizierung der Komponenten der jeweiligen Reaktionsgemische :

| | |
|---|---|
| Säule: | DB 1, 60 m, 0,32 mm, 1,0 µm |
| Gaschromatograph: | HP 5890 mit Autosampler |
| Injektortemperatur: | 250°C |
| Detektortemperatur: | 300°C |
| Temperaturprogramm: | 200°C - 15 min isotherm - 5°C/min - 280°C |
| Interner Standard: | DE GDME |
| Auswertung: | HP ChemStation |

### Beispiel 2: Hydrierung BCMI

In einem 300 mL Miniplantautoklav mit Einrichtungen zur Absicherung von Druck und Temperatur wurden 4,7 g Ra-Co 2724 der Firma Grace Division in 40 g 2-Me-THF vorgelegt. Der Autoklav wird auf 120°C hochgeheizt und Wasserstoff bis zu einem Gesamtdruck von 100 bar aufgepresst. Innerhalb von 120 min wird eine Mischung aus 18 g BCMI, 1,8 g DEGDME und 98 g 2-Me-THF zudosiert. Die Reaktionsmischung wird für weitere 60 min unter Reaktionsbedingungen gerührt. Der Austrag wird mittels Methanol homogenisiert und über GC analysiert. Folgende Selektivitäten werden gefunden: 44,5% tert.-Me-TETA, 47,2% sek.-Me-TETA, 6,4% TETA und 1,3% AEPIP.

## Patentansprüche

1. Verfahren zur Herstellung von Triethylentetraamin, das mit mindestens einer Methylgruppe substituiert ist (Me-TETA), umfassend die Hydrierung von Biscyanomethylimidazolidin (BCMI) in Gegenwart eines Katalysators.

2. Verfahren gemäß Anspruch 1, **dadurch gekennzeichnet, dass** das Me-TETA ausgewählt ist aus Triethylentetraamin, das mit einer Methylgruppe substituiert ist (mono-Me-TETA), Triethylentetraamin, das mit zwei Methylgruppen substituiert ist (bis-Me-TETA), oder Triethylentetraamin, das mit drei Methylgruppen substituiert ist (tris-Me-TETA).

3. Verfahren gemäß Anspruch 1 oder 2, **dadurch gekennzeichnet, dass** das Me-TETA ausgewählt ist aus N-2-Aminoethyl-N'-(2-N"-methylaminoethyl)-1,2-ethandiamin (sek-Me-TETA) oder N-2-Aminoethyl-N-methyl-N'-2-aminoethyl-1,2-ethandiamin (tert-Me-TETA).

4. Verfahren gemäß einem der Ansprüche 1 bis 3, **dadurch gekennzeichnet, dass** bei der Hydrierung von BCMI ein Gemisch enthaltend mindestens 2 Me-TETAs anfällt.

5. Verfahren gemäß Anspruch 4, **dadurch gekennzeichnet, dass** mindestens ein Me-TETA aus dem bei der Hydrierung anfallenden Me-TETA-Gemisch isoliert wird.

6. Verfahren gemäß einem der Ansprüche 1 bis 5, **dadurch gekennzeichnet, dass** als Katalysator ein Raney-Katalysator eingesetzt wird, bevorzugt ein Raney-Nickel- oder ein Raney-Kobalt-Katalysator, insbesondere ein Raney-Kobalt-Skelett-Katalysator, der aus einer Co/Al-Legierung durch Laugung mit wässriger Alkalimetallhydroxid-Lösung erhalten wurde und der als Promotor mindestens eines der Elemente Fe, Ni oder Cr enthält.

7. Verfahren gemäß einem der Ansprüche 1 bis 6, **dadurch gekennzeichnet, dass** die Hydrierung in Gegenwart eines Lösungsmittels, insbesondere 2-Methyl-Tetrahydrofuran (2-Me-THF), Tetrahydrofuran (THF) oder Methanol, durchgeführt wird.

8. Verfahren gemäß einem der Ansprüche 1 bis 7, **dadurch gekennzeichnet, dass** der Druck 30 bis 250 bar und/oder die Temperatur 80°C bis 140°C beträgt.

9. Verfahren gemäß einem der Ansprüche 1 bis 8, **dadurch gekennzeichnet, dass** die Hydrierung in Gegenwart eines Additivs, insbesondere in Gegenwart von Ethylendiamin (EDA) oder Ammoniak, durchgeführt wird.

10. Verfahren gemäß einem der Ansprüche 1 bis 9, **dadurch gekennzeichnet, dass** BCMI hergestellt wird durch Umsetzung von Ethylendiamindiacetonitril (EDDN) und Formaldehyd.

11. Verfahren gemäß Anspruch 10, **dadurch gekennzeichnet, dass** im BCMI-haltigen Reaktionsgemisch enthaltenes Wasser durch eine Destillation abgetrennt wird.

12. Verfahren gemäß Anspruch 11, **dadurch gekennzeichnet, dass** zur Destillation von Wasser ein Dünnschichtverdampfer, insbesondere ein Sambay, eingesetzt wird.

13. Verfahren gemäß einem der Ansprüche 10 bis 12, **dadurch gekennzeichnet, dass** EDDN hergestellt wird durch Umsetzung von EDA mit Formaldehyd und Blausäure (HCN).

14. Verfahren gemäß Anspruch 13, **dadurch gekennzeichnet, dass** die EDDN-Herstellung gemäß einer der Optionen i) bis iv) durchgeführt wird, wobei
i) Formaldehyd und HCN zunächst zu Formaldehydcyanhydrin (FACH) und anschließend Ethylendiamin (EDA) mit FACH umgesetzt wird, wobei das molare Verhältnis von EDA zu FACH 1 : 1,5 bis 1 : 2 [mol/mol] beträgt, oder
ii) ein Ethylendiamin-Formaldehyd-Addukt (EDFA) mit HCN umgesetzt wird, wobei das molare Verhältnis von EDFA zu HCN 1 : 1,5 bis 1 : 2 [mol/mol] beträgt, oder
iii) EDA mit einem Gemisch aus Formaldehyd und Blausäure (GFB) umgesetzt wird, wobei das molare Verhältnis von EDA zu GFB 1 : 1,5 bis 1 : 2 [mol/mol] beträgt, oder
iv) EDA zeitgleich mit Formaldehyd und HCN umgesetzt wird, wobei das molare Verhältnis von EDA zu Formaldehyd zu HCN 1 : 1,5 : 1,5 bis 1 : 2 : 2 [mol/mol/mol] beträgt.

## Claims

1. A process for preparing triethylenetetramine substituted by at least one methyl group (Me-TETA), comprising the hydrogenation of biscyanomethylimidazolidine (BCMI) in the presence of a catalyst.

2. The process according to claim 1, wherein the Me-TETA is selected from triethylenetetramine substituted by one methyl group (mono-Me-TETA), triethylenetetramine substituted by two methyl groups (bis-Me-TETA), or triethylenetetramine substituted by three methyl groups (tris-Me-TETA).

3. The process according to claim 1 or 2, wherein the Me-TETA is selected from N-2-aminoethyl-N'-(2-N''-methylaminoethyl)-1,2-ethanediamine (sec-Me-TETA) and N-2-aminoethyl-N-methyl-N'-2-aminoethyl-1,2-ethanediamine (tert-Me-TETA).

4. The process according to any of claims 1 to 3, wherein the hydrogenation of BCMI gives a mixture comprising at least 2 Me-TETAs.

5. The process according to claim 4, wherein at least one Me-TETA is isolated from the Me-TETA mixture obtained in the hydrogenation.

6. The process according to any of claims 1 to 5, wherein the catalyst used is a Raney catalyst, preferably a Raney nickel or Raney cobalt catalyst, especially a Raney cobalt skeletal catalyst which has been obtained from a Co/Al alloy by leaching with aqueous alkali metal hydroxide solution and which comprises, as a promoter, at least one of the elements Fe, Ni or Cr.

7. The process according to any of claims 1 to 6, wherein the hydrogenation is performed in the presence of a solvent, especially 2-methyltetrahydrofuran (2-Me-THF), tetrahydrofuran (THF) or methanol.

8. The process according to any of claims 1 to 7, wherein the pressure is 30 to 250 bar and/or the temperature is 80°C to 140°C.

9. The process according to any of claims 1 to 8, wherein the hydrogenation is performed in the presence of an additive, especially in the presence of ethylenediamine (EDA) or ammonia.

10. The process according to any of claims 1 to 9, wherein BCMI is prepared by reaction of ethylenediaminediacetonitrile (EDDN) and formaldehyde.

11. The process according to claim 10, wherein water present in the BCMI-containing reaction mixture is removed by a distillation.

12. The process according to claim 11, wherein a thin-film evaporator, especially a Sambay, is used for distillation of water.

13. The process according to any of claims 10 to 12, wherein EDDN is prepared by reacting EDA with formaldehyde and hydrogen cyanide (HCN).

14. The process according to claim 13, wherein the EDDN preparation is performed according to one of the options i) to iv), in which
i) formaldehyde and HCN are first converted to formaldehyde cyanohydrin (FACH) and then ethylenediamine (EDA) is reacted with FACH, where the molar ratio of EDA to FACH is 1 : 1.5 to 1 : 2 [mol/mol], or
ii) an ethylenediamine-formaldehyde adduct (EDFA) is reacted with HCN, where the molar ratio of EDFA to HCN is 1 : 1.5 to 1 : 2 [mol/mol], or
iii)EDA is reacted with a mixture of formaldehyde
and hydrogen cyanide (MFH), where the molar ratio of EDA to MFH is 1 : 1.5 to 1 : 2 [mol/mol], or
iv) EDA is reacted simultaneously with formaldehyde and HCN, where the molar ratio of EDA to formaldehyde to HCN is 1 : 1.5 : 1.5 to 1 : 2 : 2 [mol/mol/mol].

## Revendications

1. Procédé pour la préparation de triéthylènetétramine qui est substituée par au moins un groupe méthyle (Me-TETA), comprenant l'hydrogénation de biscyanométhylimidazolidine (BCMI) en présence d'un catalyseur.

2. Procédé selon la revendication 1, **caractérisé en ce que** la Me-TETA est choisie parmi la triéthylènetétramine qui est substituée par un groupe méthyle (mono-Me-TETA), la triéthylènetétramine qui est substituée par deux groupes méthyle (bis-Me-TETA), ou la triéthylènetétramine qui est substituée par trois groupes méthyle (tris-Me-TETA).

3. Procédé selon la revendication 1 ou 2, **caractérisé en ce que** la Me-TETA est choisie parmi la N-2-aminoéthyl-N'-(2-N''-méthylaminoéthyl)-1,2-éthane-diamine (sec-Me-TETA) ou la N-2-aminoéthyl-N-méthyl-N'-(2-aminoéthyl)-1,2-éthanediamine (tert-Me-TETA).

4. Procédé selon l'une quelconque des revendications 1 à 3, **caractérisé en ce que** lors de l'hydrogénation de la BCMI il se forme un mélange contenant au moins 2 Me-TETAs.

5. Procédé selon la revendication 4, **caractérisé en ce qu'**on isole au moins une Me-TETA à partir du mélange de Me-TETA formé lors de l'hydrogénation.

6. Procédé selon l'une quelconque des revendications 1 à 5, **caractérisé en ce qu'**on utilise comme catalyseur un catalyseur de Raney, de préférence un catalyseur au nickel de Raney ou un catalyseur au cobalt de Raney, en particulier un catalyseur à squelette cobalt de Raney, qui a été obtenu à partir d'un alliage Co/Al par lixiviation avec une solution aqueuse d'un hydroxyde de métal alcalin et qui contient comme promoteur au moins l'un des éléments Fe, Ni ou Cr.

7. Procédé selon l'une quelconque des revendications 1 à 6, **caractérisé en ce qu'**on effectue l'hydrogénation en présence d'un solvant, en particulier le 2-méthyltétrahydrofurane (2-Me-THF), le tétrahydrofurane (THF) ou le méthanol.

8. Procédé selon l'une quelconque des revendications 1 à 7, **caractérisé en ce que** la pression vaut de 30 à 250 bars et/ou la température vaut de 80 °C à 140 °C.

9. Procédé selon l'une quelconque des revendications 1 à 8, **caractérisé en ce qu'**on effectue l'hydrogénation en présence d'un additif, en particulier en présence d'éthylènediamine (EDA) ou d'ammoniac.

10. Procédé selon l'une quelconque des revendications 1 à 9, **caractérisé en ce que** la BCMI est préparée par mise en réaction d'éthylènediaminediacétonitrile (EDDN) et de formaldéhyde.

11. Procédé selon la revendication 10, **caractérisé en ce qu'**on sépare par une distillation l'eau contenue dans le mélange réactionnel contenant de la BCMI.

12. Procédé selon la revendication 11, **caractérisé en ce que** pour la distillation de l'eau on utilise un évaporateur à couche mince, en particulier un Sambay.

13. Procédé selon l'une quelconque des revendications 10 à 12, **caractérisé en ce que** l'EDDN est préparé par mise en réaction d'EDA avec du formaldéhyde et de l'acide cyanhydrique (HCN).

14. Procédé selon la revendication 13, **caractérisé en ce qu'**on effectue la préparation de l'EDDN selon l'une des options i) à iv),
i) en faisant réagir d'abord du formaldéhyde et HCN pour obtenir de la formaldéhydecyanhydrine (FACH) et ensuite de l'éthylènediamine (EDA) avec FACH, le rapport molaire d'EDA à FACH valant de 1 : 1,5 à 1 : 2 [mole/moles], ou
ii) en faisant réagir un adduit éthylèdiamine-formaldéhyde (EDFA) avec HCN, le rapport molaire d'EDFA à HCN valant de 1 : 1,5 à 1 : 2 [mole/moles], ou
iii) en faisant réagir de l'EDA avec un mélange de formaldéhyde et d'acide cyanhydrique (GFB), le rapport molaire d'EDA à GFB valant de 1 : 1,5 à 1 : 2 [mole/moles], ou
iv) en faisant réagir de l'EDA en même temps avec du formaldéhyde et HCN, le rapport molaire d'EDA à formaldéhyde à HCN valant de 1 : 1,5 : 1,5 à 1 : 2 : 2 [mole/moles/moles].
